# EUROPÄISCHE PATENTSCHRIFT

(11) **EP 3 062 877 B1**
(45) Veröffentlichungstag und Bekanntmachung des Hinweises auf die Patenterteilung: **04.12.2019**
(21) Anmeldenummer: 14796734.3
(22) Anmeldetag: 31.10.2014
(51) Int. Cl.: A61N 1/375

(54) **ELEKTRISCHE ANORDNUNG MIT EINEM IMPLANTIERBAREN KABELELEMENT**
ELECTRIC ASSEMBLY COMPRISING AN IMPLANTABLE CABLE ELEMENT
AGENCEMENT ÉLECTRIQUE COMPRENANT UN ÉLÉMENT DE CÂBLE POUVANT ÊTRE IMPLANTÉ

(30) Priorität: 31.10.2013 EP 13191130
(43) Veröffentlichungstag der Anmeldung: 07.09.2016
(73) Patentinhaber: Berlin Heart GmbH, 12247 Berlin (DE)
(72) Erfinder: ARNOLD, Mario, 12157 Berlin (DE); WINTERWERBER, Kim Peter, 12357 Berlin (DE)
(74) Vertreter: Pfenning, Meinig & Partner mbB
(86) Internationale Anmeldenummer: PCT/EP2014/073470
(87) Internationale Veröffentlichungsnummer: WO 2015/063272

(56) Entgegenhaltungen:
- WO-A1-00/57948
- WO-A1-94/01175
- WO-A1-2005/075017
- US-A1- 2013 190 551

## Beschreibung

Die Erfindung liegt auf mechanischem und elektrotechnischem Gebiet und ist mit besonderem Vorteil in der Medizintechnik anwendbar.

In der Medizintechnik ist es möglich geworden, eine Anzahl von Körperfunktionen eines Patienten durch elektrische Aggregate zu messen, zu unterstützen oder allgemein positiv zu beeinflussen. So sind seit langer Zeit Herzschrittmacher üblich, die elektrisch betrieben werden, sowie elektrische Dosierpumpen, Stimulationselemente und in jüngerer Vergangenheit auch Blutpumpen zur Unterstützung der Herzfunktion. Einige dieser elektrischen Aggregate benötigen einen Kabelanschluss und eine Verbindung über ein implantierbares Kabel zum Körperäußeren, wo die Aggregate entweder mit einer Energieversorgung oder mit einer Steuereinheit oder beidem verbindbar sind.

Da viele der genannten Aggregate über längere Zeit in einem Patientenkörper verbleiben, ergibt sich bei den üblichen Bewegungen des Patienten eine ständige Beanspruchung des implantierbaren Kabels und damit eine hohe Anforderung an dessen Standzeit. Probleme, die sich ergeben können, sind beispielsweise Leiterbruch, Verschleiß der Leiterisolierung, beispielsweise der Isolierung zwischen zwei parallel geführten Leitern, oder auch Bruch oder Abrieb eines Kabelmantels. Auch die Leiter selbst können bei dauernder Belastung brechen.

Beispielsweise beschreibt die Veröffentlichung WO 2005/075017 A1 eine perkutane Leitungsanordnung zur Übertragung elektrischer Signale an ein medizinisches Gerät, das in einem Körper eines Patienten implantiert ist. Die Leitungsanordnung umfasst ein flexibles längliches Element mit einem ersten Abschnitt, der geeignet ist, außerhalb des Körpers des Patienten zu bleiben und einen ersten Durchmesser aufweist, und einen zweiten Abschnitt, der mit dem ersten Abschnitt verbunden und angepasst ist, sich durch ein Loch in einer Hautschicht des Körpers des Patienten hindurch zu erstrecken. Der zweite Abschnitt hat einen zweiten Durchmesser, der wesentlich kleiner als der erste Durchmesser ist.

In der Veröffentlichungen WO 00/57948 A1, WO 94/01175 A1 und US 2013/0190551 A1 sind weitere Beispiele für elektrische Anordnungen mit implantierbaren Kabelelementen beschrieben.

Die vorliegende Erfindung hat sich vor dem Hintergrund des Standes der Technik die Aufgabe gestellt, eine elektrische Anordnung mit einem implantierbaren Kabelelement zu schaffen, die bei möglichst einfacher Konstruktion eine hohe Zuverlässigkeit mit einer langen Standzeit verbindet.

Die Aufgabe wird mit den Merkmalen der Erfindung gemäß Patentanspruch 1 gelöst.

Demgemäß bezieht sich ein erster Aspekt der Erfindung auf eine elektrische Anordnung mit einem implantierbaren Kabelelement, das ein im Querschnitt zentrisch angeordnetes Zugentlastungselement aufweist sowie eine um das Zugelement herum verseilte Gruppe von gegeneinander isolierten Leitern und ein gemeinsames, die elektrischen Leiter umgebendes elektrisches Abschirmelement sowie einen fluiddichten Kabelmantel.

Es ist bisher nicht üblich, für implantierbare Kabelelemente Konstruktionen mit gesonderten Zugentlastungselementen einzusetzen, wie sie beispielsweise bei Schwerlastkabeln bekannt sind. Die Erfahrung hat jedoch gezeigt, dass beispielsweise bei Herzunterstützungspumpen im Laufe der Betriebszeit relativ häufig Kabelbrüche oder Beschädigungen des Kabelmantels auftreten.

Die erfindungsgemäße Konstruktion des Kabelelements erlaubt zunächst die Trennung der elektrischen Leitungsfunktion, d. h. der Funktion, elektrische Energie oder Signale zu übertragen, einerseits und der mechanischen Stabilisierungsfunktion, die durch das Zugentlastungselement übernommen wird, andererseits. Die Querschnittsgestaltung des Kabels sieht das Zugentlastungselement im Zentrum des Querschnitts des Kabelelements vor, das weitgehend spannungsneutral ist, so dass das Zugentlastungselement beispielsweise als massives strangförmiges Bauteil ausgebildet sein kann. Das Zugentlastungselement kann allerdings auch seinerseits aus verseilten Elementen bestehen. Eine Verseilung von Leitern, die um das Zugentlastungselement herum im Idealfall symmetrisch verteilt sind, schafft eine Biegsamkeit des Kabelelements in allen Richtungen, ohne dass überhöhte Zug- oder Druckbelastungen auf die Leiter wirken. Je kürzer die Schlagweite der Verseilung gewählt wird, umso leichter ist das Kabelelement biegsam. Die einzelnen Leiter können beispielsweise zur elektrischen Energieversorgung eines Motors einer Pumpe dienen (beispielsweise drei Leiter) sowie zur Versorgung eines magnetischen Lagers (beispielsweise zwei Leiter) und beispielsweise auch zur Versorgung einer Pumpenelektronik (zwei Leiter). Zusätzlich können Leiter zur Kommunikation vorgesehen sein. Die einzelnen Leiter können jeweils aus einer oder mehreren Gruppe von ihrerseits beispielsweise ebenfalls verseilten Litzen bestehen. Die Litzen können ihrerseits beispielsweise in verschiedenen konzentrischen Schichten aufgebaut sein, die jeweils gegensinnig verseilt sind. Damit werden auftretende Tangentialkräfte bei Biegung eines Leiters kompensiert.

Die einzelnen Leiter sind jeweils mit einer Leiterisolierung versehen, wobei das Material der Isolierung derart gewählt ist, dass die Haftreibung der Leiterisolierungen gegeneinander minimiert ist. Ein geeignetes Material hierfür ist beispielsweise Polytetrafluorethylen (PTFE).

Auch das Zugentlastungselement kann mit einer PTFE-Schicht umgeben, beispielsweise mit einem PTFE-Band bandagiert sein, um ein gutes Gleiten der Leiter an dem Zugentlastungselement zu ermöglichen.

Insgesamt ist es sinnvoll, den Kabelaufbau im Querschnitt symmetrisch zu gestalten und auch Teile des Kabels, wie beispielsweise einzelne Leiter, in sich symmetrisch aufzubauen.

Als Material für die Leiter, speziell die einzelnen Litzendrähte der Leiter, kann eine Kupferlegierung eingesetzt werden oder ein Aufbau aus einem Silberkern mit einer Außenschicht aus einer Nickel-Kobalt-Basislegierung. Um Korrosionsvorgänge innerhalb des Kabelelements zu minimieren, kann jeder einzelne Leiter in seinem Außenbereich galvanisch mit einer Silber- oder Goldschicht abgedeckt werden.

Besondere Vorteile der Erfindung werden realisiert, indem das Kabelelement im Rahmen der elektrischen Anordnung mit einem implantierbaren elektrischen Aggregat, insbesondere einer Blutpumpe, mechanisch und elektrisch fest verbunden ist.

In diesem Bereich wird bewusst auf eine Steckverbindung verzichtet, um dort eine hohe Zuverlässigkeit der Verbindung mit einer leichten Abdichtbarkeit und optimalen elektrischen Abschirmung der Verbindung realisieren zu können. Die Verbindung zwischen dem Kabelelement und dem Aggregat, insbesondere der Blutpumpe, wird vor der Implantation hergestellt. In der Praxis ergibt sich selten ein Vorteil darin, ausschließlich das elektrische Aggregat ohne das implantierbare Kabelelement austauschen zu können. Das Kabelelement selbst soll durch die Erfindung derart stabil ausgeführt sein, dass an ihm Schäden während der erwarteten Betriebszeit nicht auftreten können. Der Kabelmantel des Kabelelements ist vorteilhaft mit dem Gehäuse des Aggregats durch Verschweißen, Vergießen oder Verkleben fluiddicht verbunden. Die Leiter sind vorteilhaft mit Leitern einer Durchführung des Aggregatgehäuses durch Klemmen oder Löten verbunden.

Vorteilhaft kann zudem vorgesehen sein, dass das implantierbare Kabelelement ein Steckverbindungselement zur Verbindung mit einem zweiten Kabelelement aufweist.

Das Steckverbindungselement ist üblicherweise außerhalb des implantierten Bereichs des Kabelelements vorgesehen, so dass nach Implantierung ein weiteres Kabelelement ohne Probleme mit dem implantierbaren Kabelelement verbindbar und von diesem elektrisch auch wieder lösbar ist. Wenn in diesem Bereich ein Schaden auftritt, kann dieser auch ohne einen operativen Eingriff beim Patienten behoben werden.

Die Erfindung kann außerdem vorteilhaft dadurch ausgestaltet werden, dass der Kabelmantel des implantierbaren Kabelelements abschnittsweise eine das Einwachsen von Gewebe begünstigende Schicht aufweist und dass insbesondere diese Schicht in Längsrichtung des Kabelendes von einem Steckverbindungselement beabstandet ist.

Der Kabelmantel des Kabelelements kann im implantierbaren Bereich aus einem Kunststoff bestehen. Die wichtigsten Anforderungen sind Biokompatibilität sowie mechanische Stabilität und Abriebfestigkeit. Im nicht implantierbaren Bereich des Kabelelements soll dieses zudem eine hohe UV-Beständigkeit aufweisen.

In Frage kommende Materialien sind beispielsweise Polyurethane und Silikone. Im nichtimplantierbaren bzw. -implantierten Bereich kann das implantierbare Kabelelement beispielsweise mittels eines Textilüberzugs geschützt werden.

Der das Einwachsen von Gewebe begünstigende Bereich kann eine aufgeraute, veloursartige Oberfläche aufweisen.

Zur weiteren Stabilisierung kann das Materials des Kabelmantels auch als Kompositmaterial mit einer Armierung beispielsweise durch zugfeste Aramidfasern, Glasfasern oder ein Metalldrahtgeflecht ausgestaltet werden.

Üblicherweise ist zwischen den im Querschnitt zentrisch innen angeordneten Leitern des implantierbaren Kabelelements und dem Kabelmantel eine elektrische Schirmung vorgesehen, die beispielsweise als Geflecht aus Metalldrähten, beispielsweise versilberten Kupferdrähten, oder als ein metallisiertes Kunststoffvlies ausgebildet sein kann. Besonders vorteilhaft kann auch eine Kombination aus einem metallisierten Kunststoffvlies und einem Metallgewebe verwendet werden. Auch ein Metalldrahtgeflecht im Kabelmantel kann als Schirmung dienen. Mit einer optimierten Auswahl der Schirmung kann die sogenannte Transferimpedanz des Kabelelementes optimiert werden.

Ein Aspekt der Erfindung sieht vor, dass insbesondere die das Einwachsen von Gewebe begünstigende Schicht in Längsrichtung des Kabelendes von einem Steckverbindungselement beabstandet ist.

Um das Einwandern von Keimen in den Patientenkörper im Bereich der Durchführung des Kabelelements durch die Haut zu minimieren, ist es sinnvoll, ein möglichst gutes Verwachsen des Körpergewebes mit dem Kabelmantel zu erreichen. Hierzu sind die Aspekte der Topographie der Oberfläche (Porosität, Mikrostruktur), der Elastizität der Oberfläche und einer Funktionalisierung der Oberfläche wichtig. Durch eine strukturierte Oberfläche, die beispielsweise durch Poren strukturiert ist, ergibt sich eine erhöhte Wahrscheinlichkeit der Zellanhaftung, so dass das Einwachsen begünstigt wird. Zudem begünstigt eine geringe Elastizität der Kabelmanteloberfläche das Anwachsen von Zellen.

Als besonders vorteilhaft hat sich bei geringem Konstruktionsaufwand die Konzentration auf die Strukturierung der Oberfläche herausgestellt, um eine gute Biegsamkeit zu erhalten, da die Elastizität des Kabelmantels nicht frei wählbar ist. Zusätzliche Funktionalisierungen der Manteloberfläche sind denkbar.

Um das Wandern von Keimen entlang der Kabelmanteloberfläche zu verhindern, ist vorgesehen, dass der strukturierte Bereich des Kabelmantels, der eine das Einwachsen von Gewebe begünstigende Schicht darstellt, nur wenig über den Längenbereich des Kabelelements hinausgeht, der zum Implantieren vorgesehen ist, und jedenfalls in axialer Richtung vor dem Steckverbindungselement endet und von diesem beabstandet ist. In einer bevorzugten Variante ragt der strukturierte Bereich nicht über den zur Implantation vorgesehenen Längenbereich des Kabelmantels hinaus, so dass die das Einwachsen von Gewebe begünstigende Schicht vollständig innerhalb des Körpers angeordnet werden kann.

Die Erfindung kann zudem vorteilhaft dadurch ausgestaltet werden, dass das Zugentlastungselement aus Metall, insbesondere aus Edelstahl, besteht.

Es ist auch eine Variante des Zugentlastungselements aus Aramidfasern oder anderen zugfesten Fasern, insbesondere Kunststofffasern möglich. Weitere Fasermaterialien können beispielsweise Carbon oder Dyneema oder Fasermischungen aus den drei vorgenannten Fasern sein. Edelstahl verbindet eine hohe Zugfestigkeit mit einer geringen Anfangsdehnung. Zudem ist die Anbindung des Zugentlastungselements an das elektrische Aggregat einerseits und ein Steckverbindungselement andererseits an den Enden des Kabelelements bei der Verwendung von Edelstahl besonders einfach. Hochfeste Kunststofffasern haben eine vergleichbare Zugfestigkeit und sind zudem weniger korrosionsanfällig und flexibler als Edelstahl.

Das Zugentlastungselement kann vorteilhaft an einem Gehäuse des elektrischen Aggregats, insbesondere an einer Gehäusedurchführung, befestigt sein.

Bei einer Blutpumpe, jedoch auch bei einem anderen elektrischen Aggregat, kann beispielsweise eine fluiddichte Durchführung für die elektrischen Leiter gleichzeitig als Befestigungselement für das Zugentlastungselement dienen. Eine solche Durchführung kann beispielsweise aus in Glas oder Harz eingegossenen Metallpins bestehen, auf die jeweils Crimphülsen an den Enden der Leiter des Kabelelements aufgesteckt werden können. Der Glas- oder Harzkörper der Durchführung kann zusätzlich dazu dienen, in ihn ein Ende des Zugentlastungselements einzugießen oder einzukleben und damit das Zugentlastungselement an der Durchführung und am Gehäuse des Aggregats zu befestigen.

Das Zugentlastungselement kann zur Verbesserung der Haftung an wenigstens einem, insbesondere an beiden seiner Enden Einkerbungen oder Erhöhungen an seinem mantelseitigen Umfang aufweisen.

Eine weitere vorteilhafte Ausgestaltung der Erfindung kann vorsehen, dass die Leiter des implantierbaren Kabelelements an wenigstens einem ihrer Enden mit je einer Crimphülse versehen sind, die auf Pins einer Gehäusedurchführung und/oder eines Steckverbindungselements aufsteckbar sind. In einer weiteren Ausführungsform sind die mit einer Crimphülse versehenen Enden (dann "weibliche" Enden) vorzugsweise jeweils in einen Kanal einer Gehäusedurchführung und/oder in ein Steckverbindungselement einsteckbar. In einigen Ausführungsbeispielen ist die Gehäusedurchführung ein Isolierkörper. Im Falle des Einsteckens der Enden werden zur Vervollständigung der Steckverbindung lediglich noch die männlichen Stecker in die korrespondierenden Kanäle der Gehäusedurchführung eingesteckt.

Insbesondere dann, wenn die Leiter aus einer Menge von Litzendrähten bestehen, können diese im Zuge einer Quetschverbindung mit einer Crimphülse verbunden werden, wobei die Crimphülse mit einer auf einen Kontaktpin aufsteckbaren Steckhülse verbunden sein kann. Es ergibt sich damit eine mechanische und elektrische Verbindung der Leiter jeweils mit einem Steckverbindungselement. Im Bereich der Verbindung des Kabelelements mit dem elektrischen Aggregat können die Crimphülsen vorteilhaft auch mit Pins an der Durchführung verlötet oder auf diese aufgecrimpt sein.

Ein zweiter Aspekt der Erfindung sieht vor, dass der Kabelmantel des implantierbaren Kabels eine in seiner Längsrichtung verlaufende Markierung sowie eine weitere Markierung zwischen dem Steckverbindungselement und dem Ende der das Einwachsen von Gewebe begünstigenden Schicht aufweist. Auf diese Weise ist beim Implantieren des Kabelelements für den Operateur sichtbar, wann das Kabelelement spannungsfrei im Gewebe liegt, ohne unnötig verdrillt zu sein. Dies zeigt die in Längsrichtung verlaufende Markierung an dem Kabelmantel.

In einer Ausführungsform des zweiten Aspekts ist die weitere Markierung eine quer zur Längsrichtung verlaufende Markierung, welche auf einer bestimmten definierten Länge des Kabels für den Operateur ersichtlich macht, wie weit das Kabel implantiert werden soll und wie weit beispielsweise die das Einwachsen von Gewebe begünstigende Schicht am Kabelelement reicht, soweit dies nicht unmittelbar durch Gestaltung dieser Schicht sichtbar ist.

Durch die Quermarkierung wird gewährleistet, dass die das Einwachsen begünstigende Schicht nicht aus dem Körper gezogen wird, sondern vollständig im Körper verbleibt. Auf diese Weise wird die Infektionsgefahr stark reduziert. Von daher liegt in der Quermarkierung des Kabelelements unabhängig vom internen Aufbau des Kabelelementes eine eigenständige Erfindung, welche mit den verschiedenen Aufbauten des Kabelelementes, wie in dieser Anmeldung dargestellt, kombinierbar ist. D.h. eine Quermarkierung in Verbindung mit einem Kabelelement, welches einen Abschnitt einer das Einwachsen begünstigende Schicht umfasst, wobei die Quermarkierung von diesem Abschnitt beabstandet ist, bildet eine eigenständige Erfindung. Bei der Implantation des Kabelelementes wird dieses im sterilen Zustand implantiert und anschließend an einem außerhalb des Körpers liegendem Ende entweder derart gezogen bis die Quermarkierung aus dem Körper tritt oder bei der Implantation darauf geachtet, dass die Quermarkierung im Wesentlichen im Bereich des Austritts des Kabelelementes aus dem Körper zu Liegen kommt.

In einer weiteren Ausführungsform des zweiten Aspekts umfasst die in Längsrichtung verlaufende und/oder die weitere Markierung eine Beschriftung bzw. besteht aus einer Beschriftung. Die Beschriftung kann beispielsweise Informationen beinhalten, wie weit eine das Einwachsen begünstigende Schicht noch im Patienten liegt. Hierdurch kann das Kabelelement aus dem Patienten gezogen werden, ohne dass die das Einwachsen begünstigende Schicht aus dem Körper gezogen wird. Auf diese Weise kann beispielsweise das Infektionsrisiko für den Patienten verringert werden. Auch die Markierung in der Längsrichtung kann durch eine Beschriftung gegeben sein.

In anderen Ausführungsformen weisen die Markierung oder die weitere Markierung grafische Elemente auf, wie beispielsweise eine vorbestimmte Farbgebung oder geometrische Symbole oder Linien. Die grafischen Elemente dienen dabei beispielsweise zur Information, wie weit das das Einwachsen begünstigende Gewebe noch von der Durchführungsöffnung im Körper liegt. So kann vermieden werden, dass die das Einwachsen begünstigende Schicht nicht versehentlich aus dem Körper gezogen wird.

In manchen Ausführungsformen weisen die Markierung oder die weitere Markierung haptische Elemente auf, wie beispielsweise Erhebungen oder Vertiefungen im Kabelmantel. Auch diese Elemente können einen Abstand zwischen der Markierung und der das Einwachsen des Gewebes begünstigenden Schicht repräsentieren.

Obgleich in den vorhergehenden Beispielen stets eine Markierung in Längsrichtung und eine weitere Markierung diskutiert wurde, sind Ausführungsbeispiele mit nur einer der beiden Markierungen ebenfalls von der Erfindung umfasst. Dabei können die Markierungen wie obenstehend diskutiert realisiert sein. Ebenfalls kann die elektrische Anordnung ohne eine das Einwachsen begünstigende Schicht ausgebildet sein. Des Weiteren kann der Kabelmantel durchgehend mit einer das Einwachsen begünstigenden Schicht beaufschlagt sein oder aus einem das Einwachsen begünstigenden Material bestehen oder dieses aufweisen.

Ebenfalls kann die Markierung oder die weitere Markierung einen Abstand zwischen der Markierung und einem bestimmten Punkt des Kabelmantels repräsentieren. Auf diese Weise kann eine Länge des Kabelmantels, welche im Patientenkörper verbleiben soll, zuverlässig bestimmt und ein zu weites Herausziehen des Kabels verhindert werden.

Die Erfindung bezieht sich zudem auf eine elektrische Anordnung mit einem zweiten Kabelelement, das ein erstes Steckverbindungselement zur Verbindung mit dem implantierbaren Kabel aufweist, sowie mit einer Steuereinheit zur Ansteuerung des implantierbaren elektrischen Aggregats, das mittels einer zweiten Steckverbindung mit dem zweiten Kabel verbunden ist.

Das Konzept, ein elektrisches Aggregat, ein implantierbares Kabelelement, ein zusätzliches extrakorporales Kabelelement und eine Steuereinheit über zwei lösbare Steckverbindungen, einerseits im Bereich zwischen dem implantierbaren Kabelelement und dem extrakorporalen Kabelelement, andererseits zwischen dem extrakorporalen Kabelelement und dem Steuerelement zu verbinden, stellt aus Sicht der Erfindung das Optimum an Zuverlässigkeit und Haltbarkeit einerseits und praktischer Handhabbarkeit andererseits dar. Das extrakorporale Kabel (zweite Kabel) und die Steuereinheit sind dadurch leicht austauschbar. Bei der Anpassung an einen Patienten kann über die Längenauswahl des extrakorporalen Kabels die Gesamtlänge der Kabelverbindung zwischen Steuereinheit und Aggregat angepasst werden. Zudem kann das extrakorporale Kabel, das besonders hohen mechanischen Belastungen durch Knicken und Abrieb ausgesetzt ist, ohne Probleme und Anpassungsaufwand ausgetauscht werden.

Im Folgenden wird die Erfindung anhand eines Ausführungsbeispiels in Zeichnungen gezeigt und nachfolgend erläutert. Dabei zeigt
- Fig. 1: im Überblick schematisch das Zusammenwirken eines implantierbaren Kabelelements der elektrischen Anordnung mit einem elektrischen Aggregat, einem weiteren Kabelelement und einer Steuereinheit,
- Fig. 2: das implantierbare Kabelelement im Querschnitt,
- Fig. 3: eine Seitenansicht des implantierbaren Kabelelements mit einem Steckverbindungselement,
- Fig. 4: die Steckverbindung des implantierbaren Kabelelements mit einem weiteren Kabelelement in einer schematischen Ansicht,
- Fig. 5: die Anbindung des implantierbaren Kabelelements an das elektrische Aggregat
- Fign. 6a-6d: verschiedene Varianten der Anbindung des Zugentlastungselements an ein Aggregat sowie
- Fign. 7a-d: verschiedene Varianten von Markierungen.

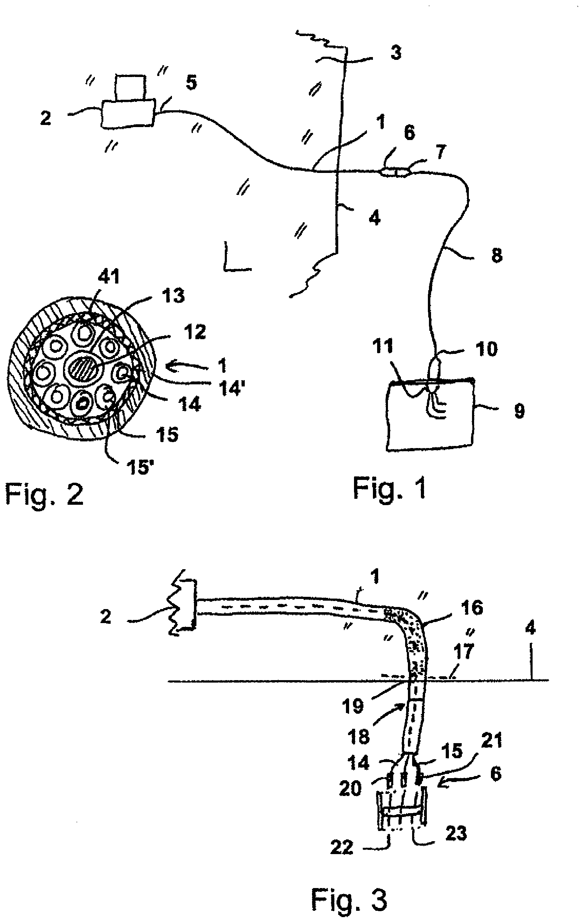

**Figur 1** zeigt schematisch ein implantierbares Kabelelement 1, das gemeinsam mit einem elektrischen Aggregat 2 in Form einer Blutpumpe in dem Körper 3 eines Patienten implantiert ist. Die Linie 4 zeigt dabei im Querschnitt die Hautoberfläche des Patienten, d. h. die Oberfläche und Trennfläche zwischen dem Körperinneren und dem Körperäußeren, an. Das implantierbare Kabelelement 1 ist dabei an seinem körperinneren Ende 5 mit dem Aggregat 2 fest verbunden. Damit ist gemeint, dass in diesem Bereich keine Steckverbindung vorgesehen ist, sondern dass die einzelnen Elemente des implantierbaren Kabelelements 1 mit Teilen des Aggregats 2 durch Fügeverbindungen, wie Schweißen, Kleben, Löten, Klemmen oder dergleichen, verbunden sind. Auf die Arten der Verbindung wird weiter unten noch detaillierter eingegangen.

An seinem dem Aggregat 2 entfernteren Ende weist das implantierbare Kabelelement 1 ein Steckverbindungselement 6 zur Herstellung einer Steckverbindung 6, 7 mit einem weiteren Steckverbindungselement 7 eines zweiten Kabelelements 8 auf. Das zweite Kabelelement 8 verbindet das implantierbare Kabelelement 1 mit einer Steuereinheit 9. Dazu weist das zweite Kabelelement 8 an seinem Ende 10, das der Steuereinrichtung zugewandt ist, ein drittes Steckverbindungselement zum Einstecken in eine gehäusefeste Buchse 11 des Steuerelements 9 auf. Die elektrische Anordnung kommt somit insgesamt mit zwei Steckverbindungen 6, 7 und 10, 11 aus. Dies führt dazu, dass das zweite Kabelelement 8, das besonders beansprucht ist, problemlos ausgetauscht werden kann.

In **Figur 2** ist im Detail im Querschnitt der Aufbau des ersten implantierbaren Kabelelements 1 gezeigt. Dieses weist zentral ein Zugentlastungselement 12 auf, das beispielsweise aus hochfesten Kunststoffasern wie Aramid besteht. Das Zugentlastungselement 12 kann mit einer Lage 13 von Bandagen aus einer Kunststofffolie umwickelt sein, um das Gleiten der zentrisch um das Zugentlastungselement 12 herum angeordneten Leitern 14, 15 zu erleichtern. Die Leiter 14, 15, von denen um das Zugentlastungselement 12 herum im dargestellten Beispiel acht vorhanden sind, sind jeweils einzeln mit einer Isolierung 14', 15' umgeben, wobei die Isolierungen außer der elektrischen Isolierung der Leiter gegeneinander auch ein leichtes Gleiten gegeneinander bei einem Biegen oder Bewegen des Kabelelements bewirken.

Die Leiter 14, 15 sind um das Zugentlastungselement 12 herum verseilt um eine besondere Flexibilität des Kabels zu erreichen. Jeder einzelne der Leiter 14, 15 besteht aus einer Gruppe von Litzendrähten, die ihrerseits in einer oder mehreren konzentrischen Schichten verseilt sein können. Einzelne Litzendrähte können dabei insofern inhomogen sein, als sie aus einem zentralen Silberkern mit einer äußeren Beschichtung aus einer Nickel-Kobalt-Basislegierung bestehen. Es wäre alternativ dazu allerdings auch die Herstellung aus einer hochfesten Kupferlegierung möglich. Werden in anderen Ausführungsbeispielen die Leiter mehrlagig angeordnet, können die Leiter der verschiedenen Lagen beispielsweise mit wechselndem Schlag angeordnet werden.

Die Leiter insgesamt können als Litzenbündel mit einer Silber- oder Goldschicht durch Galvanisieren versehen sein, um einerseits physikalisch eine Abdeckung des Leitermaterials zu bieten und andererseits durch die besondere Stellung der Edelmetalle in der elektrochemischen Spannungsreihe einer Korrosion vorzubeugen.

Die verseilte Gruppe von Leitern 14, 15 ihrerseits ist durch eine Kombination aus einem metallisierten Kunststoffvlies und einem Geflecht von Kupferlitzen radial außen abgedeckt, die eine elektrische Schirmung 41 bilden. Das Litzengeflecht kann dabei grundsätzlich als Armierung des Kabelmantels in diesen integriert und eingegossen sein. Es kann jedoch auch radial innerhalb des Kabelmantels vorgesehen sein.

Um die Anforderungen an den Kabelmantel bezüglich UV-Beständigkeit, Biokompatibilität, Abriebfestigkeit und mechanische Eigenschaften sowie der Elastizität zu erfüllen, kommen Materialien wie Polyurethane und Silikone in Betracht. Für diese ist eine Armierung durch Verstärkungselemente, wie Drahtlitzen, Glasfasern und Ähnliches, sowie eine Abdeckung durch eine Textilschicht möglich.

In **Figur 3** ist schematisch die gesamte Länge des implantierbaren Kabelelements 1 dargestellt, von dem elektrischen Aggregat 2 beginnend bis zu dem Steckverbindungselement 6. Es ist dargestellt, dass in dem Bereich, in dem das Kabelelement 1 durch die Haut 4 des Patienten hindurchtritt, das Äußere des Kabelmantels mit einer das Ein- oder Anwachsen von lebendem Gewebe begünstigenden Außenschicht ausgestattet ist, die in Fig. 3 gepunktet dargestellt und mit 16 bezeichnet ist. Die Schicht 16 erstreckt sich bis zu einem axialen Bereich 17 der Kabellänge, der durch eine gestrichelte Linie dargestellt ist, d. h., der Bereich 16 ist in axialer Richtung von dem Steckverbindungselement 6 beabstandet, erstreckt sich also nicht bis zum Steckverbindungselement 6. Dies verhindert, dass sich entlang der Kabellänge außerhalb des Patientenkörpers Keime an dem äußeren Kabelmantel festsetzen, deren Anwachsen und Vermehrung durch eine aufgeraute Struktur der Oberfläche des Kabelmantels begünstigt würde. Zudem sind in Fig. 3 optische Markierungen dargestellt, von denen die erste Markierung 18 als in Umfangsrichtung um den Kabelmantel umlaufende Linie ausgeführt ist, die für den Operateur sichtbar einen Bereich kennzeichnet, der außerhalb des Patientenkörpers liegen muss.

Zudem ist als weitere Markierung 19 eine gestrichelte Linie dargestellt, die sich in axialer Richtung achsparallel zur Längsachse des Kabelelements entlang des Kabelmantels des implantierbaren Kabelelements erstreckt. Wenn das implantierbare Kabelelement 1 verdreht ist, so ist dies für einen Operateur durch das helixartige Umlaufen der gestrichelten Linie 19 leicht sichtbar, und er kann die Spannung, die durch das Verdrehen des Kabelelements 1 entstanden ist, durch entsprechendes Zurückdrehen beseitigen. Dies begünstigt eine problemlose dauerhafte Lagerung des Kabelelements 1 im Körper eines Patienten.

An dem zweiten Ende des implantierbaren Kabelelements 1 ist dargestellt, dass die einzelnen Leiter 14, 15 in Crimphülsen 20, 21 aufgenommen sind, die auf Kontaktpins 22, 23 eines Steckverbindungselements 6 aufsteckbar und dort verklemmbar oder verlötbar sind. Das Steckverbindungselement 6 kann dann mit einem Steckverbindungselement 7 eines weiteren Kabelelements außerhalb des Patientenkörpers zusammengesteckt werden.

Die Crimphülsen 20, 21 sind mit den Leitern 14, 15 durch Quetschen oder Löten mechanisch und elektrisch fest verbunden.

In einem weiteren Ausführungsbeispiel kann das Steckverbindungselement 6 als Isolierkörper ausgebildet sein. In diesem Fall weist der Isolierkörper Kanäle auf, in welche die mit den Crimphülsen 20, 21 versehenen Leiter 14 und 15 eingesteckt werden. Die Kanäle sind als durch den Isolierkörper verlaufende Öffnungen mit einem ersten und einem zweiten Ende ausgebildet, wobei die Leiter 14 und 15 in das erste Ende jeweils eines Kanals eingeführt werden. In diesem Ausführungsbespiel werden zur Anbindung der Steckverbindung männliche Pins in das zweite Ende der Kanäle eingeführt, so dass die männlichen Pins die Leiter 14 und 15 jeweils kontaktieren.

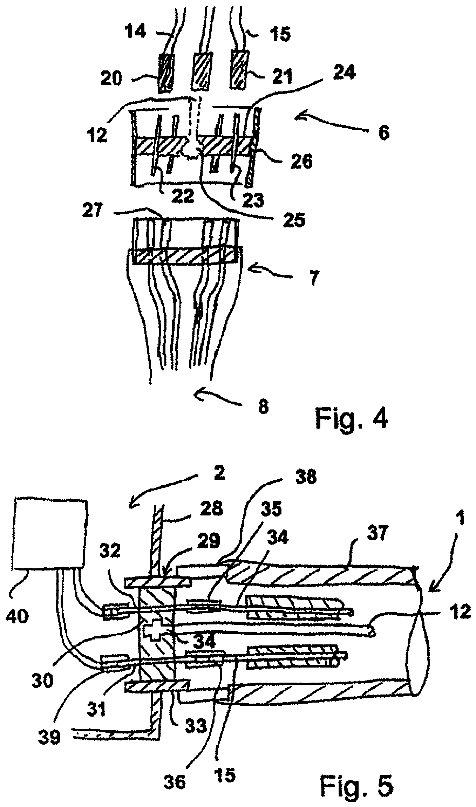

**Figur 4** zeigt in vergrößertem Maßstab nochmals die Enden der Leiter 14, 15 des implantierbaren Kabelelements 1 mit aufgecrimpten Endhülsen 20, 21, die auf Kontaktpins 22, 23 des Steckverbindungselements 6 aufsteckbar sind. Die Pins 22, 23 sind in einen Isolierträger 24 eingegossen, der beispielsweise aus einem Harz bestehen kann und in den ebenfalls ein Ende 25 des Zugentlastungselements 12 mit einer mantelseitigen Verdickung oder Vertiefung eingegossen ist. Der Isolierträger 24 ist in eine Steckerhülse 26 eingegossen oder eingeklebt. In diese Steckerhülse und auf das Steckverbindungselement 6 steckbar ist das Steckverbindungselement 7, das sich am Ende des zweiten Kabelelements 8 befindet. Dort sind entsprechende Steckhülsen 27 mit den einzelnen Leitern des zweiten Kabelelements 8 verbunden und in dem Steckverbindungselement 7 fixiert, die auf die Kontaktpins 22, 23 aufsteckbar sind.

Das Kabelelement 8 kann beispielsweise einen Aufbau aufweisen, der dem Aufbau des implantierbaren Kabelelements 1 ganz oder teilweise entspricht.

In **Figur 5** ist die Anbindung des implantierbaren Kabelelements 1 an das Gehäuse, genauer an eine Durchführung 29 an dem Gehäuse 28 eines elektrischen Aggregats, insbesondere einer Herzpumpe, dargestellt. In der Gehäusewand 28 ist die Durchführung 29 dargestellt, die eine Hülse 33 und einen in die Hülse gasdicht eingepassten Glaskörper 30 aufweist. In den Glaskörper 30 sind durchgehende Kontaktpins 31, 32 fluiddicht eingegossen. Ein Ende 34 des Zugentlastungselements 12, das Hinterschneidungen aufweist, ist in den Glaskörper 30 eingegossen, um dort die Anbindung der Zugkräfte an das Gehäuse des Aggregats zu gewährleisten. Hierdurch wird das implantierbare Kabelelement 1 wirksam zugentlastet. Die Leiter 14, 15 des Kabelelements 1 sind in endseitigen Kontakthülsen 35, 36 durch Crimpen befestigt, wobei die Kontakthülsen auf die durchgehenden Kontaktpins 31, 32 aufgeschoben und mit diesen beispielsweise verlötet sein können. Der Kabelmantel 37, der die äußerste Schicht des Kabelelements 1 darstellt, ist am Ende mit einer Überwurfhülse 38 in Form einer Überwurfmutter oder einer Bajonetthülse verbunden, die ihrerseits fluiddicht mit der Hülse 33 verbunden ist, beispielsweise durch Kleben oder Löten. Auf der Innenseite des Gehäuses 28 sind die durchgehenden Kontaktpins 31, 32 über Kontaktstellen, beispielsweise ebenfalls in Form von Crimphülsen 39, mit den Anschlüssen eines elektrischen Elements, beispielsweise eines Motors 40, verbunden.

Die dargestellten Leiter 14, 15 sind nur beispielhaft dargestellt, und die Erfindung ist so zu verstehen, dass die Zahl der Leiter sowie die übertragenen Potentiale und Signale vielfältig gestaltet sein können. Im Falle einer Blutpumpe können außer Leitern für die elektrische Energieversorgung des Motors 40 zudem Leiter zur Versorgung eines magnetischen Lagers und zur Übermittlung von Signalen vorgesehen sein.

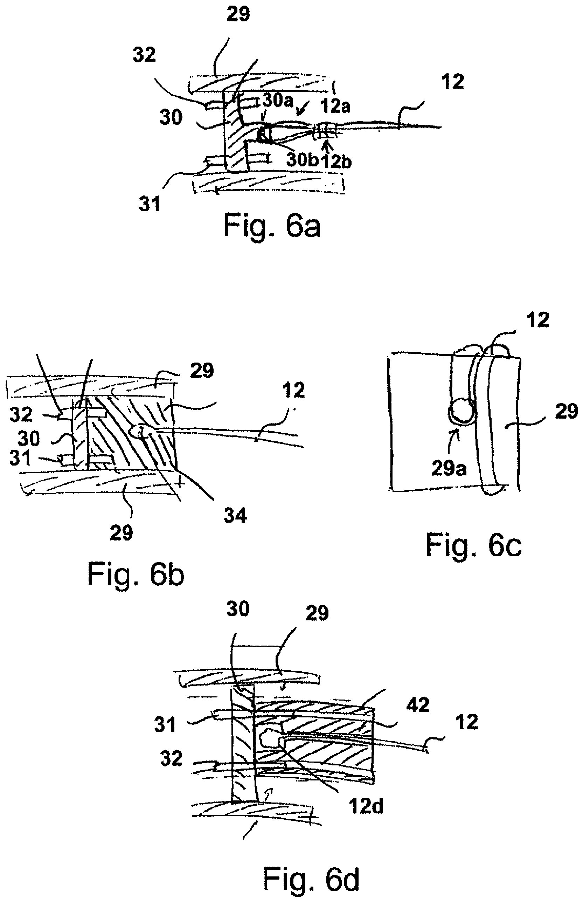

In den **Figuren 6a bis 6d** sind verschiedene Varianten zur Befestigung des Zugentlastungselements 12 mit dem Aggregat 2 dargestellt. Hierbei wird jeweils auf eine detaillierte Beschreibung des Aggregats verzichtet. Details zum Aggregat können den vorangehenden Beschreibungen entnommen werden.

In der Figur 6a ist eine Durchführung 29 dargestellt, welche den Glaskörper 30 mit Kontaktpins 31 und 32 zeigt. Zudem umfasst der Glaskörper 30 einen sich vom Aggregat weg erstreckenden Steg 30a mit einer Bohrung 30b, welche eine Öffnungsweite besitzt, die eine Durchführung der Aramidfaser 12 ermöglichst. Das Zugentlastungselement 12 ist dabei derart ausgebildet, dass es eine Schlaufe 12a umfasst, welche durch eine Crimphülse 12b fixiert ist. Vor dem Aufbringen der Crimphülse 12b wird die Schlaufe 12a durch die Bohrung 30b gefädelt und die Schlaufe anschließend mit der Crimphülse 12b fixiert. Alternativ zur Verwendung der Crimphülse 12b kann die Schlaufe auch verknotet werden.

In der Figur 6b ist eine Durchführung 29 mit einer Glasdurchführung 30 und Pins 31 und 32 dargestellt. Das Zugentlastungselement 12 weist an seinem dem Aggregat zugewandten Ende eine Verknotung 12c auf, so dass eine lokale Verdickung des Zugentlastungselements 12 an seinem Ende stattfindet. Das Zugentlastungselement 12 ist beispielsweise mit einem Polyamid vergossen, so dass das Zugentlastungselement 12 sicher mit der Glasdurchführung verbunden ist. Dabei werden die Pins zuerst aufgesteckt und die Faser ausgerichtet, vorzugsweise die Faser im Anschluss an das Aufstecken der Pins ausgerichtet, und anschließend die Vergussmasse eingefüllt.

Zur Herstellung des Vergusses 34 wird das Ende des Zugentlastungselements 12 im Bereich der Glasdurchführung angeordnet und anschließend die Vergussmasse eingefüllt. Nach dem Aushärten kann die Durchführung mit dem Aggregat verbunden werden. Alternativ ist die Durchführung bereits mit dem Aggregat verbunden und das Vergießen des Zugentlastungselements findet anschließend statt.

In der Figur 6c ist eine weitere Alternative zur Verbindung des Zugentlastungselements 12 mit der Durchführung 29 dargestellt. In der Figur 6c ist die Außenseite der Durchführung 29 dargestellt. Diese umfasst eine Bohrung 29a, welche auf der dem Aggregat abgewandten Seite der Glasdurchführung angeordnet ist. Durch diese Bohrung wird ein Ende des Zugentlastungselements 12 gezogen und dieses wird anschließend auf der Außenseite entweder verknotet oder mit einer Crimphülse versehen, so dass bei Zug auf das Zugentlastungselement das Garn sich fester um die Durchführung 29 zieht.

Eine weitere Alternative ist in der Figur 6d dargestellt. Hierbei weist die Durchführung 29 nebst der Glasdurchführung 30 und den Pins 31 und 32 einen weiteren Isolierkörper 42 auf, welcher an seiner dem der Glasdurchführung zugewandten Seite eine Öffnung umfasst, durch welche ein Zugentlastungselement 12 gefädelt werden kann, wobei ein Knoten 12d des Zugentlastungselements ein Herausrutschen des Zugentlastungselements in einer dem Aggregat abgewandten Richtung verhindert. Der Isolierkörper wird dabei auf die Glasdurchführung gesteckt und mit aus dem Stand der Technik bekannten Methoden mit diesem verbunden.

Die Merkmale der Erfindung erlauben den Aufbau eines implantierbaren Kabelelements sowie einer entsprechenden elektrischen Anordnung, die wenigstens ein Kabelelement und gegebenenfalls zusätzliche Elemente enthält, in einer Weise, die für den Patienten eine hohe Zuverlässigkeit mit einer langen Standzeit und einem hohen Anwendungskomfort verbindet.

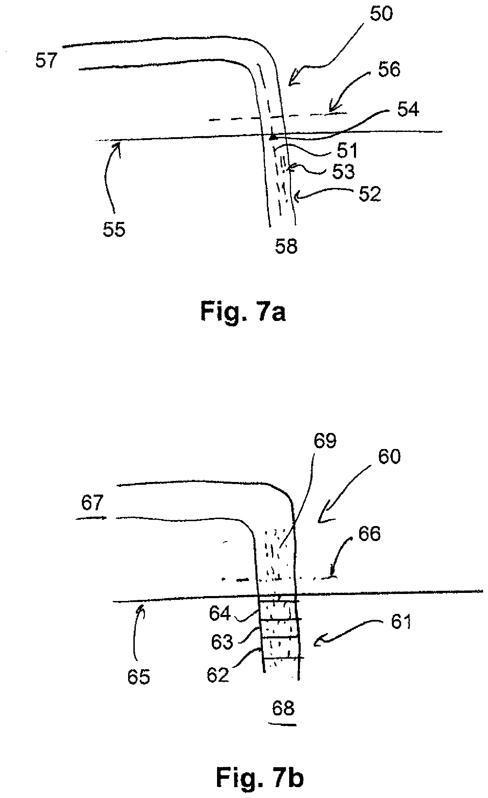

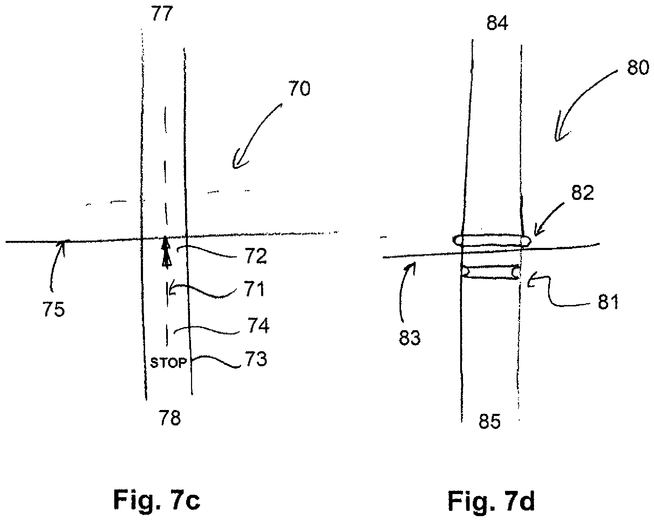

Anhand der **Figuren 7** sollen verschiedenen Varianten von Markierungen näher erläutert werden.

In Figur 7a ist ein implantierbares Kabelelement bzw. ein sichtbarer Kabelmantel 50 gezeigt, welcher eine Längsmarkierung 51 und weitere Markierungen 52 bis 54 umfasst. Das Kabelelement 50 durchstößt die Haut 55 im Bereich der dargestellten Markierung 54. Mithilfe der Markierung 54 soll vermieden werden, dass das Kabelelement 50 weiter als bis zum eingezeichneten Bereich 56 des Kabelelements 50 herausgezogen wird. Dabei befindet sich das distale Ende des Kabelelements im Körper und das proximale Ende 58 außerhalb des Körpers. Bei der weiteren Markierung 54 handelt es sich um ein Dreieck, welches sich vom proximalen zum distalen Ende des Kabelelements hin verjüngt. Die Spitze des Dreiecks stellt dabei eine Markierung dar, über welche das Kabelelement nicht herausgezogen werden sollte. Damit ein Operateur nicht durch die Markierung überrascht werden kann, sind weitere Markierungen 52 und 53 auf dem Kabelelement angeordnet, welche beispielsweise im Falle der Markierung 52 ein Ausrufezeichen und im Falle der Markierung 53 ein doppeltes Ausrufezeichen sein können. Diese Markierungen warnen den Operateur, dass demnächst die für die Lage des Kabelelements entscheidende Markierung 54 sichtbar werden wird.

Das eingezeichnete Kabelelement weist keine das Einwachsen begünstigende Schicht auf, kann jedoch auch eine das Einwachsen begünstigende Schicht umfassen, welche beispielsweise bis zur Schnittebene 56 reicht. Selbstverständlich ist auch ein Kabelelement 50, welches vollständig mit einer das Einwachsen begünstigenden Schicht überdeckt ist, möglich. Die weitere Markierung 54 definiert hier einen Abstand zur Schnittebene 56.

In Figur 7b ist ein weiteres Ausführungsbeispiel verschiedener Markierungen dargestellt. Das Kabelelement 60 umfasst ein Markierungssystem 61, welches über Markierungen 62 bis 64 verfügt. Eine explizite Längsmarkierung ist nicht vorhanden, kann aber alternativ auch vorhanden sein. Die Markierungen 62, 63 und 64 sind in unterschiedlichen Farben gehaltene Bänder. Auch hier dienen die Bänder dazu, einen Abstand zu einer Schnittebene 66 zu markieren. Dadurch soll gewährleistet werden, dass die Schnittebene 66 nicht durch die Haut 65 gezogen wird. Es ist erkennbar, wie vom distalen Ende 68 zum proximalen Ende 67 hin eine das Einwachsen begünstigende Schicht 69 vorhanden ist. Diese erstreckt sich sowohl innerhalb als auch außerhalb des Körpers. Daher definieren die farbigen Bänder lediglich einen Abstand zur Schnittebene 66.

Eine weitere Ausführungsform eines Kabelelements ist in Figur 7c dargestellt. Das Kabelelement 70 weist eine Längsmarkierung 71 auf, mithilfe welcher eine Verdrillung erkannt werden kann. Des Weiteren ist eine Doppelpfeilspitze 72 vorhanden, welche einen Abstand zu einer vordefinierten Schnittebene anzeigt. Weiterhin ist eine Beschriftung 73 erkennbar, welche das Wort "STOP" umfasst, um so einen Operateur auf den nahen Doppelpfeil hinzuweisen. Sobald die Spitze des Doppelpfeils die Haut 75 aus dem Körperinneren kommend verlassen hat, kann der Operateur einen Zug auf das Seil beenden, da das Kabelelement 70 seine gewünschte Lage erreicht hat. Das Kabelelement 70 besitzt an seinem proximalen Ende 77 gegebenenfalls eine das Einwachsen begünstigende Schicht. Diese ist jedoch jenseits der eingezeichneten Schnittebene bis zum distalen Ende 78 hin nicht vorhanden.

Eine weitere Ausführungsform ist in Figur 7d dargestellt. Das Kabelelement 80 umfasst hierbei Markierungen 81 und 82, welche als haptische Elemente ausgebildet sind. Dabei ist die Markierung 81 beispielsweise als umlaufende Vertiefung im Kabelelement 80 ausgebildet. Diese Markierung weist darauf hin, dass eine wulstförmige Markierung 82, welche im Körper verbleiben soll, in einem vorbestimmten Abstand von der Markierung 81 entfernt liegt. Auf diese Weise wird gewährleistet, dass der Wulst 82 nicht durch die Haut 83 tritt, und es wird ferner ein übermäßiger Zug auf das distale Ende 84 vermieden. Das proximale Ende 85 liegt außerhalb des Körpers.

### Bezugszeichenliste

- 1: implantierbares Kabelelement
- 2: Aggregat
- 3: Körper
- 4: Haut
- 5: erstes Ende von 1
- 6: Steckverbindungselement
- 7: weiteres Steckverbindungselement
- 8: zweites Kabelelement
- 9: Steuereinheit
- 10: Ende des zweiten Kabelelements
- 11: gehäusefeste Steckbuchse
- 12: Zugentlastungselement
- 13: Lage von Bandagen
- 14, 15: Leiter
- 14', 15': Leiterisolierung
- 16: Bereich des Kabelmantels mit Einwachshilfe
- 17: Ende von 16
- 18: Markierung (quer)
- 19: Markierung (längs)
- 20, 21: Crimphülsen
- 22, 23: Kontaktpins von 6
- 24: Isolierträger
- 25: Ende
- 26: Steckerhülse
- 27: Steckerhülse
- 28: Gehäuse eines Aggregats
- 29: Durchführung
- 30: Glaskörper
- 31: Kontaktpins von 29
- 32: Kontaktpins von 29
- 33: Hülse
- 34: Ende von 12
- 35, 36: Kontakthülsen
- 37: Kabelmantel
- 38: Überwurfhülse
- 39: Crimphülse
- 40: Motor
- 41: Abschirmung

## Patentansprüche

1. Elektrische Anordnung mit einem implantierbaren Kabelelement (1), das eine Gruppe von gegeneinander isolierten Leitern (14, 15) und einen fluiddichten Kabelmantel (37) aufweist und der Kabelmantel (37) eine in seiner Längsrichtung verlaufende Markierung (19) sowie auch eine weitere Markierung (18) zwischen einem Steckverbindungselement (6) und einem Ende einer das Einwachsen von Gewebe begünstigenden Schicht (16) aufweist, wobei das Steckverbindungselement zur Verbindung mit einem zweiten Kabelelement (8) angeordnet ist, wobei die in Längsrichtung verlaufende Markierung (19) ein grafisches Element aufweist, beispielsweise in Form einer vorbestimmten Farbgebung, eines geometrischen Symbols, oder einer Linie, oder ein haptisches Element aufweist, das als Erhebung oder Vertiefung des Kabelmantels ausgebildet ist, wobei sich die in Längsrichtung verlaufende Markierung in axialer Richtung achsparallel zur Längsachse des Kabelelements (1) entlang des Kabelmantels (37) des implantierbaren Kabelelements (1) erstreckt.

2. Elektrische Anordnung nach Anspruch 1, **dadurch gekennzeichnet, dass** die weitere Markierung eine quer verlaufende Markierung ist.

3. Elektrische Anordnung nach einem der Ansprüche 1 oder 2, **dadurch gekennzeichnet, dass** die Markierung oder weitere Markierung eine Beschriftung umfasst.

4. Elektrische Anordnung nach einem der vorhergehenden Ansprüche, **dadurch gekennzeichnet, dass** die Markierung oder die weitere Markierung mindestens einen Abstand zwischen der Markierung bzw. weitere Markierung und dem Ende der das Einwachsen von Gewebe begünstigende Schicht (16) repräsentiert.

5. Elektrische Anordnung nach einem der vorangehenden Ansprüche, **dadurch gekennzeichnet, dass** die weitere Markierung (18) eine den Kabelmantel (37) umlaufende Linie umfasst, die für den Operateur sichtbar einen Bereich kennzeichnet, der außerhalb eines Patientenkörpers liegen soll.

6. Elektrische Anordnung nach einem der vorangehenden Ansprüche, **dadurch gekennzeichnet, dass** die weitere Markierung (18) ein Dreieck umfasst ist, welches sich vom proximalen zum distalen Ende des Kabelelements hin verjüngt, wobei eine Spitze des Dreiecks eine Markierung darstellt, über welche das Kabelelement nicht aus einem Patientenkörper herausgezogen werden soll.

7. Elektrische Anordnung nach einem der vorhergehenden Ansprüche, **dadurch gekennzeichnet, dass** die Markierung oder weitere Markierung eine haptische Markierung oder eine farbliche Markierung ist.

8. Elektrische Anordnung nach einem der vorhergehenden Ansprüche, **dadurch gekennzeichnet, dass** das implantierbare Kabelelement (1) ein im Querschnitt zentrisch angeordnetes Zugentlastungselement (12) aufweist und die Gruppe von gegeneinander isolierten Leitern (14, 15) um das Zugentlastungselement herum verseilt ist und ein gemeinsames, die elektrischen Leiter (14, 15) umgebendes elektrisches Abschirmelement (41) vorhanden ist.

9. Elektrische Anordnung nach Anspruch 8, **dadurch gekennzeichnet, dass** das implantierbare Kabelelement (1) mit einem implantierbaren elektrischen Aggregat (2), insbesondere einer Blutpumpe, mechanisch und elektrisch fest verbunden ist.

10. Elektrische Anordnung nach einem der vorhergehenden Ansprüche, **dadurch gekennzeichnet, dass** der Kabelmantel (37) des implantierbaren Kabelelements (1) abschnittsweise das das Einwachsen von Gewebe begünstigende Schicht (16) aufweist und dass insbesondere diese Schicht in Längsrichtung des Kabelendes vomSteckverbindungselement (6) beabstandet ist.

11. Elektrische Anordnung nach einem der Ansprüche 8 bis 10, **dadurch gekennzeichnet, dass** das Zugentlastungselement (12) aus Kunststofffasern besteht.

12. Elektrische Anordnung nach einem der Ansprüche 8 bis 11, **dadurch gekennzeichnet, dass** das Zugentlastungselement (12) an einem Gehäuse (28) des elektrischen Aggregats (2), insbesondere an einer Gehäusedurchführung (29), befestigt ist.

13. Elektrische Anordnung nach Anspruch 12, **dadurch gekennzeichnet, dass** ein Ende (34) des Zugentlastungselements (12) in eine Gehäusedurchführung (29) eingegossen oder angebunden ist.

14. Elektrische Anordnung nach einem der vorhergehenden Ansprüche, **dadurch gekennzeichnet, dass** die Leiter (14,15) des implantierbaren Kabelelements (1) an wenigstens einem ihrer Enden mit je einer Crimphülse (20, 21, 35, 36) versehen sind, die auf Pins (22, 23, 31, 32) einer Gehäusedurchführung (29) und/oder eines Steckverbindungselements (6) aufsteckbar oder in die Pins (22, 23, 31, 32) und/oder das Steckverbindungselement (6) einsteckbar sind.

15. Elektrische Anordnung nach Anspruch 9, **dadurch gekennzeichnet, dass** ein Steckverbindungselement (7) zur Verbindung mit dem implantierbaren Kabel (1) aufweist, sowie mit einer Steuereinheit (9) zur Ansteuerung des implantierbaren elektrischen Aggregats (2), das mittels einer zweiten Steckverbindung (10, 11) mit dem zweiten Kabel verbunden ist.

## Claims

1. An electrical arrangement with an implantable cable element (1) comprising a group of conductors (14, 15) which are insulated with respect to one another, and a fluid-tight cable sheath (37), and the cable sheath (37) comprises a marking (19) running in its longitudinal direction as well as a further marking (18) between a plug-in connection element (6) and an end of a layer (16) promoting the ingrowth of tissue, wherein the plug-in connection element is arranged for connection to a second cable element (8), wherein the marking (19) running in the longitudinal direction comprises a graphic element, for example in the form of a predefined colour, a geometric symbol, or a line, or comprises a haptic element configured as a protrusion or indentation of the cable sheath, wherein the marking running in the longitudinal direction axially extends parallel to the longitudinal axis of the cable element (1) along the cable sheath (37) of the implantable cable element (1).

2. An electrical arrangement according to claim 1, **characterised in that** the further marking is a transversely running marking.

3. An electrical arrangement according to one of claims 1 or 2, **characterised in that** the marking or further marking comprises an inscription.

4. An electrical arrangement according to one of the preceding claims, **characterised in that** the marking or the further marking represents at least a distance between the marking or the further marking respectively, and the end of the layer (16) promoting the ingrowth of tissue.

5. An electrical arrangement according to one of the preceding claims, **characterised in that** the further marking (18) comprises a circumferential line on the cable sheath (37) which indicates to a surgeon an area that is to be on the outside of a patient's body.

6. An electrical arrangement according to one of the preceding claims, **characterised in that** the further marking (18) comprises a triangle which tapers from the proximal end of the cable element to the distal end, wherein an apex of the triangle represents a marking beyond which the cable element is not to be pulled out of a patient's body.

7. An electrical arrangement according to one of the preceding claims, **characterised in that** the marking or the further marking is a haptic marking or a coloured marking.

8. An electrical arrangement according to one of the preceding claims, **characterised in that** the implantable cable element (1) comprises a strain relief element (12) which is arranged centrically in the cross section, and the group of conductors (14, 15) which are insulated with respect to one another is stranded around the strain relief element, and a common electrical shielding element (41) which surrounds the electrical conductors (14, 15) is present.

9. An electrical arrangement according to claim 8, **characterised in that** the implantable cable element (1) is mechanically and electrically connected in a fixed manner to an implantable electrical assembly (2), in particular a blood pump.

10. An electrical arrangement according to one of the preceding claims, **characterised in that** the cable sheath (37) of the implantable cable element (1) in sections comprises the layer (16) promoting the ingrowth of tissue and that in particular this layer is distanced to the plug-in connection element (6) in the longitudinal direction of the cable end.

11. An electrical arrangement according to one of the claims 8 to 10, **characterised in that** the strain relief element (12) consists of plastic fibres.

12. An electrical arrangement according to one of the claims 8 to 11, **characterised in that** the strain relief element (12) is fastened on a housing (28) of the electrical assembly (2), in particular on a housing feed-through (29).

13. An electrical arrangement according to claim 12, **characterised in that** an end (34) of the strain relief element (12) is moulded into or connected onto a housing feed-through (29).

14. An electrical arrangement according to one of the preceding claims, **characterised in that** the conductors (14, 15) of the implantable cable element (1) are provided at least at one of their ends in each case with a crimp sleeve (20, 21, 35, 36) which can be stuck onto pins (22, 23, 31, 32) of a housing feed-through (29) and/or of a plug-in connection element (6) or can be stuck into the pins (22, 23, 31, 32) and/or the plug-in connection element (6).

15. An electrical arrangement according to claim 9, **characterised in that** it comprises a plug-in connection element (7) for connection to the implantable cable (1), as well as comprising a control unit (9) for controlling the implantable electrical assembly (2) which is connected to the second cable by way of a second plug-in connection (10, 11).

## Revendications

1. Dispositif électrique doté d'un élément de câble (1) implantable qui présente un groupe de conducteurs (14, 15) isolés les uns des autres et une gaine de câble (37) étanche aux fluides, et la gaine de câble (37) présente un marquage (19) s'étendant dans sa direction longitudinale ainsi qu'un autre marquage (18) entre un élément connecteur (6) et une extrémité d'une couche (16) favorisant l'incarnation de tissu, où l'élément connecteur est disposé pour la connexion avec un deuxième élément de câble (8), où le marquage (19) s'étendant dans la direction longitudinale présente un élément graphique, par exemple, sous la forme d'une coloration prédéterminée, d'un symbole géométrique, ou d'une ligne ou un élément haptique, qui est conçu sous forme d'une excroissance ou d'une cavité de la gaine de câble, où le marquage s'étendant dans la direction longitudinale se déploie dans la direction axiale de manière parallèle à l'axe par rapport à l'axe longitudinal de l'élément de câble (1) le long de la gaine de câble (37) de l'élément de câble (1) implantable.

2. Dispositif électrique selon la revendication 1, **caractérisé en ce que** l'autre marquage est un marquage s'étendant de manière perpendiculaire.

3. Dispositif électrique selon la revendication 1 ou la revendication 2, **caractérisé en ce que** le marquage, ou l'autre marquage, comprend une impression.

4. Dispositif électrique selon l'une des revendications précédentes, **caractérisé en ce que** le marquage, ou l'autre marquage, représente au moins un espace entre le marquage, respectivement l'autre marquage, et l'extrémité de la couche (16) favorisant l'incarnation de tissu.

5. Dispositif électrique selon l'une des revendications précédentes, **caractérisé en ce que** l'autre marquage (18) comprend une ligne entourant la gaine de câble (37) qui caractérise de manière visible pour l'opérateur une zone qui doit se situer en dehors du corps du patient.

6. Dispositif électrique selon l'une des revendications précédentes, **caractérisé en ce que** l'autre marquage (18) comprend un triangle, lequel s'amenuise à partir de l'extrémité proximale vers l'extrémité distale de l'élément de cathéter, où une pointe du triangle représente un marquage au-delà duquel l'élément de câble ne doit pas être retiré du corps du patient.

7. Dispositif électrique selon l'une des revendications précédentes, **caractérisé en ce que** le marquage, ou l'autre marquage, est un marquage haptique ou un marquage de couleur.

8. Dispositif électrique selon l'une des revendications précédentes, **caractérisé en ce que** l'élément de câble (1) implantable présente un élément de réduction de la tension (12) disposé de manière centrale dans la section transversale et le groupe de conducteurs (14, 15) isolés les uns des autres est encordé autour de l'élément de réduction de la tension et un élément de blindage (41) électrique commun entourant les conducteurs (14, 15) électriques est présent.

9. Dispositif électrique selon la revendication 8, **caractérisé en ce que** l'élément de câble (1) implantable est relié solidement de manière mécanique et électrique avec un ensemble (2) électrique implantable, notamment une pompe à sang.

10. Dispositif électrique selon l'une des revendications précédentes, **caractérisé en ce que** la gaine de câble (37) de l'élément de câble (1) implantable présente par endroits la couche (16) favorisant l'incarnation de tissu et que cette couche est en particulier espacée de l'élément connecteur (6) en direction de l'extrémité de câble.

11. Dispositif électrique selon l'une des revendications 8 à 10, **caractérisé en ce que** l'élément de réduction de la tension (12) est constitué de fibres de matière plastique.

12. Dispositif électrique selon l'une des revendications 8 à 11, **caractérisé en ce que** l'élément de réduction de tension (12) est fixé sur le boîtier (28) de l'ensemble (2) électrique, notamment sur un passage de boîtier (29).

13. Dispositif électrique selon la revendication 12, **caractérisé en ce qu'**une extrémité (34) de l'élément de réduction de la tension (12) est coulée ou attachée dans un passage de boîtier (29).

14. Dispositif électrique selon l'une des revendications précédentes, **caractérisé en ce que** les conducteurs (14, 15) de l'élément de câble (1) implantable sont munis chacun d'un manchon de sertissage (20, 21, 35, 36) au moins au niveau de leurs extrémités, qui peuvent être piqués sur des broches (22, 23, 31, 32) d'un passage de boîtier (29) et/ou d'un élément connecteur (6), ou peuvent être piqués dans les broches (22, 23, 31, 32) et/ou dans l'élément connecteur (6).

15. Dispositif électrique selon la revendication 9, **caractérisé en ce qu'**il présente un élément connecteur (7) pour la connexion avec le câble (1) implantable ainsi qu'avec une unité de commande (9) pour contrôler l'ensemble (2) électrique implantable qui est reliée avec le deuxième câble au moyen d'un deuxième connecteur (10, 11).
